# EUROPEAN PATENT SPECIFICATION

(11) **EP 0 810 002 B1**
(45) Date of publication and mention of the grant of the patent: **16.07.2003**
(21) Application number: 97201581.2
(22) Date of filing: 29.05.1997
(51) Int. Cl.: A61M 25/00

(54) **Suction catheter with preformed tip**
Absaugkatheter mit vorgeformter Spitze
Cathéter d'aspiration avec pointe préformée

(30) Priority: 29.05.1996 NL 1003226
(43) Date of publication of application: 03.12.1997
(73) Proprietor: Cordis Europa N.V., 9301 LJ Roden (NL)
(72) Inventor: Reekers, Jan Albertus, 1185 CR Amstelveen (NL)
(74) Representative: 't Jong, Bastiaan Jacob

(56) References cited:
- EP-A- 0 351 206
- EP-A- 0 693 295
- US-A- 5 037 403

## Description

The invention relates to a suction catheter comprising a tube-like basic body with a proximal and a distal end inside of which at least a discharge lumen and a further lumen extends, wherein a suction inlet connected to the discharge lumen has been arranged inside the basic body at a distance from the distal end, and a preformed, curved and pliable tip extends the distal end.

Such a suction catheter is known from EP-A-0 351 206. This known catheter is suitable for withdrawing, injecting or monitoring fluids within the central vasculature. The curved and pliable tip forms a blunt service, preventing damage to the blood vessel wall and preventing the introduction of the catheter in small vessels.

The object of the invention is to further improve a catheter of this known kind.

According to the invention, this object is achieved with a suction catheter of the kind set forth above, having the characterizing features of claim 1. In this way, the catheter can be used for instance to remove blood thrombi from the blood stream of a patient. The curved and pliable tip facilitates using the suction catheter also when larger blood vessels, such as the pulmonary artery, are concerned.

An advantageous further development has been characterised in claim 2. This measure prevents the suction inlet from attaching itself to the wall of a blood vessel. The preformed curved tip ensures that the suction inlet is kept at a certain distance from the wall of the blood vessels.

It has been found that the suction catheter as characterised in claim 3 is easy to handle.

Preferably the measure as set out in claim 4 is employed. The catheter can then be guided by means of a guide wire.

The measure as set out in claim 5 can in addition be employed in an advantageous manner. In that case the guide-wire-lumen extends substantially only in the end-section, so that in the section of the basic body extending from the proximal end to the suction inlet no space is taken up by the guide-wire-lumen. Consequently the discharge lumen can be given a maximum cross-section.

The invention will be explained in greater detail below, with reference to the suction catheter according to the invention as illustrated in the figures.
- Figure 1: illustrates a partly broken away view of a suction catheter according to an embodiment of the invention.
- Figure 2: shows an enlarged detail indicated by arrow II in figure 1.
- Figure 3: illustrates a cross-section along the line III-III in figure 2.
- Figure 4: illustrates schematically the use of the suction catheter.

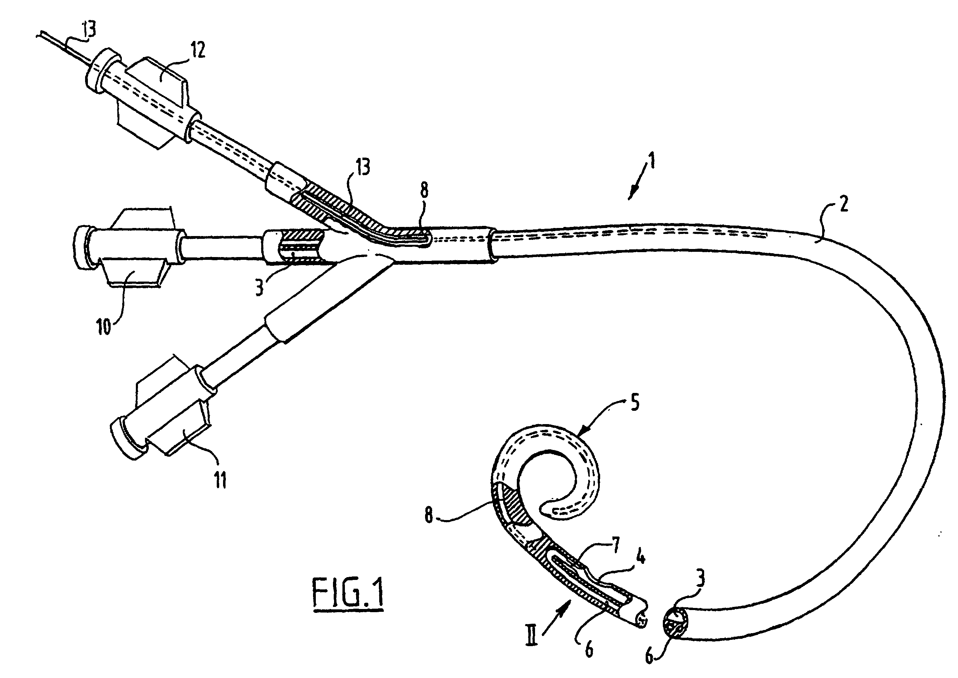

The catheter 1 illustrated in figure 1 comprises a basic body 2, in which, at a distance from the distal end, a suction inlet 4 has been formed. This suction inlet 4 is connected with a discharge lumen, which extends form this opening 4 in the proximal direction to the proximal end of the catheter. There the discharge lumen 3 opens into a discharge connection 10. From the suction inlet 4, a preformed, curved and pliable tip 5 extends in the distal direction to the distal end. The curve is of the pigtail-type.

As can be seen in the figure, the tip 5 is curved in the direction in which the suction inlet 4 is pointing. As a result the suction inlet 4 is at all times kept at a certain distance from the wall of a blood vessel into which the catheter has been introduced. The catheter shown in figure 1 is a preferred embodiment wherein the suction action at the suction inlet 4 is effected by means of ejector action. A pressure lumen 6 has been arranged inside the basic body 2 which ends close to the relatively distal end of the suction inlet 4, that is to say on the left-hand side in figure 1, in a jet nozzle 7 which is directed in the opposite, proximal direction along the suction inlet in the discharge lumen 3.

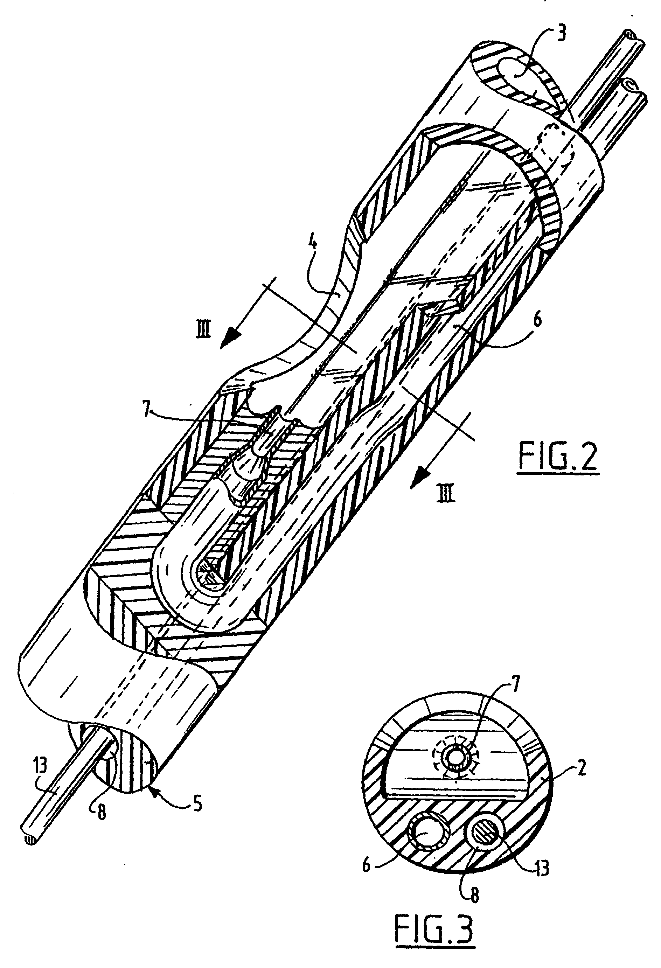

As can be seen in greater detail in figure 2, a fluid jet can be directed along the opening 4 by means of the jet nozzle 7, as a result of which suction will be generated at the suction inlet 4 due to the ejector action. The material sucked in will be passed along by the liquid released from the jet nozzle 7 through the discharge lumen 3 to the discharge connection 10.

At the proximal end the pressure lumen 6 is connected to a pressure connection 11.

For the purpose of introducing the catheter 1, the curved tip 5 can be straightened by using a guide wire 13, which is advanced through a guide-wire-lumen 8 arranged for that purpose inside the basic body 2. At the proximal end this guide- wire-lumen 8 is connected with a guide-wire-connection 12.

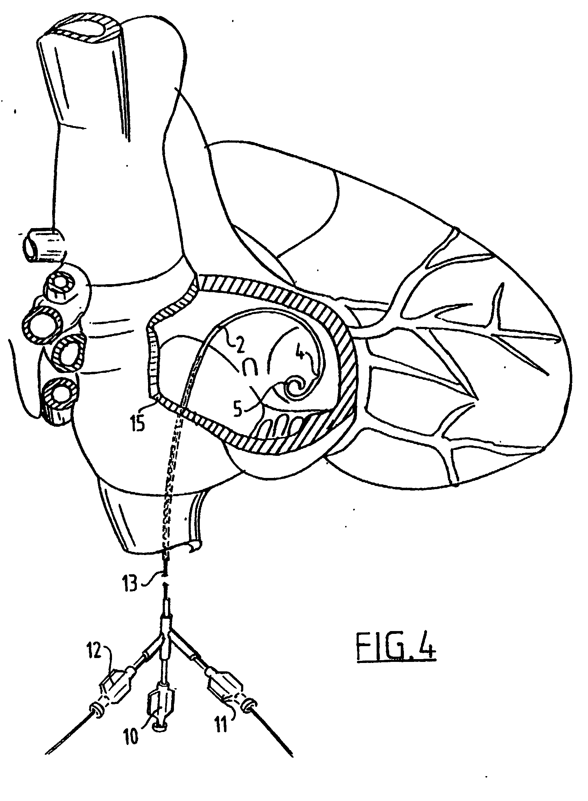

It should be noted that the connections 10, 11 and 12 in the figures 1 and 4 have only been illustrated schematically. A catheter used for the purpose described will have been provided at the proximal end with haemostatic devices in order to prevent undesired leaking out of blood via the lumens of the catheter.

As can be seen in figure 4, the catheter 1 with the curved preformed tip 5 can be employed in large blood vessels and even be advanced through the heart 15 into the pulmonary artery. Because of the preformed Curve, the suction inlet 4 is positioned in a stable manner as required. The suction inlet 4 cannot attach itself to the wall as it is kept, by means of the curve 5, at a certain distance from the walls of the blood vessel.

Although the guide-wire-lumen in the example of an embodiment shown extends right from the proximal end to the distal end, the catheter according to the invention can also be given the embodiment of a 'rapid exchange'-type. In that case the guide-wire-lumen extends from an opening in the wall of the basic body 2 positioned at a limited distance from the distal end to the distal end. With such an embodiment the guide-wire-lumen can be formed in the end-section only in a suitable manner. In that case the guide-wire-lumen does not take up any space in the section of the basic body through which the discharge lumen 3 and the pressure lumen 6 extend. These can in that case be given an optimum cross-section for the purpose of ejector action.

The tip may be connected to the remaining part of the catheter in any suitable manner, for instance by means of glueing or welding. The specific shape of the curve may be adapted to the required operative position. The tip may have been manufactured in a suitable manner of a plastic material opaque to X-rays, so that the tip can be made properly visible on an X-ray screen.

## Claims

1. Suction catheter comprising a tube-like basic body (2) with a proximal and a distal end inside of which at least a discharge lumen (3) and a further lumem (6) extends, wherein a suction inlet (4) connected to the discharge lumen (3) has been arranged inside the basic body (2) at a distance from the distal end, and a preformed, curved and pliable tip (5) extends the distal end, **characterized in that** the tip (5) extends from the suction inlet to the distal end and **in that** the further lumen (6) is a pressure lumen extending inside the basic body (2) which ends, close to the relatively distal end of the suction inlet, in a jet nozzle (7) directed in a proximal direction along the suction inlet (4) in the discharge lumen (3).

2. Suction catheter as claimed in claim 1, wherein the pliable tip (5) has been curved in a preformed manner in the direction in which the suction inlet (4) is pointing.

3. Suction catheter as claimed in claim 1 or 2, wherein the preformed curve is of the pigtail-type.

4. Catheter as claimed in one of the previous claims, wherein the discharge lumen (3) extends to the suction inlet (4) and a guide-wire-lumen (8) extends to the distal end.

5. Suction catheter as claimed in claim 4, wherein the guide-wire-lumen extends from an opening positioned distal to the suction inlet.

## Patentansprüche

1. Absaugkatheter, umfassend einen röhrenförmigen Grundkörper (2) mit einem proximalen und einem distalen Ende, in dem sich mindestens ein Ausstoßlumen (3) und ein weiteres Lumen (6) erstrecken, wobei ein mit dem Ausstoßlumen (3) verbundener Absaugeinlass (4) im Inneren des Grundkörpers (2) bei einem Abstand vom distalen Ende angeordnet wurde, und wobei eine vorgeformte, gebogene und biegsame Spitze (5) am distalen Ende verläuft, **dadurch gekennzeichnet, dass** die Spitze (5) sich vom Absaugeinlass bis zum distalen Ende erstreckt, und das das weitere Lumen (6) ein im Inneren des Grundkörpers (2) verlaufendes Drucklumen ist, welches in der Nähe des relativ distalen-Endes des Absaugeinlasses in einer Strahldüse (7) endet, die in eine Proximalrichtung entlang des Absaugeinlasses (4) im Ausstoßlumen (6) gerichtet ist.

2. Absaugkatheter wie im Anspruch 1 beansprucht, wobei die biegsame Spitze (5) auf eine vorgeformte Weise in die Richtung gebogen wurde, in die der Absaugeinlass (4) zeigt.

3. Absaugkatheter wie im Anspruch 1 oder 2 beansprucht, wobei der vorgeformte Bogen einer Art Schweineschwanz entspricht.

4. Katheter wie in einem der vorangehenden Ansprüche beansprucht, wobei das Ausstoßlumen (3) sich zum Absaugeinlass (4) hin erstreckt und ein Führungsdraht-Lumen (8) sich zum distalen Ende hin erstreckt.

5. Absaugkatheter wie im Anspruch 4 beansprucht, wobei das Führungsdraht-Lumen (8) sich von einer Öffnung aus erstreckt, die distal zum Absaugeinlass positioniert ist.

## Revendications

1. Cathéter d'aspiration comprenant un corps de base ressemblant à un tube (2) avec une extrémité proximale et une extrémité distale dans lesquelles au moins une lumière d'écoulement (3) et une autre lumière (6) s'étendent, dans lequel un orifice d'aspiration (4) connecté à la lumière d'écoulement (3) a été disposé à l'intérieur du corps de base (2) à une distance de l'extrémité distale, et une extrémité préformée, recourbée et pliable (5) prolonge l'extrémité distale, **caractérisé en ce que** l'extrémité (5) s'étend de l'orifice d'aspiration jusqu'à l'extrémité distale et **en ce que** l'autre lumière (6) est une lumière sous pression s'étendant dans le corps de base (2) qui se termine, près de l'extrémité relativement distale de l'orifice d'aspiration, en une buse d'éjection (7) dirigée dans une direction proximale le long de l'orifice d'aspiration (4) dans la lumière d'écoulement (3).

2. Cathéter d'aspiration selon la revendication 1, dans lequel l'extrémité pliable (5) a été recourbée d'une manière préformée dans la direction dans laquelle l'orifice d'aspiration (4) pointe.

3. Cathéter d'aspiration selon la revendication 1 ou 2, dans lequel la courbure préformée est du type en forme de queue de cochon ou pigtail.

4. Cathéter selon l'une quelconque des revendications précédentes, dans lequel la lumière d'écoulement s'étend jusqu'à l'orifice d'aspiration et une lumière de fil guide (8) s'étend jusqu'à l'extrémité distale.

5. Cathéter d'aspiration selon la revendication 4, dans lequel la lumière du fil guide (8) s'étend d'une ouverture en position distale jusqu'à l'orifice d'aspiration.
